# EUROPÄISCHE PATENTSCHRIFT

(11) **EP 0 337 309 B1**
(45) Veröffentlichungstag und Bekanntmachung des Hinweises auf die Patenterteilung: **05.01.1994**
(21) Anmeldenummer: 89106138.4
(22) Anmeldetag: 07.04.1989
(51) Int. Cl.: C04B 35/10, C04B 35/64, A61K 6/06

(54) **Verfahren zur Herstellung von orthodontischen Teilen**
Process for the production of orthodontic parts
Procédé de fabrication de supports de correction orthodontique

(30) Priorität: 09.04.1988 DE 3811902
(43) Veröffentlichungstag der Anmeldung: 18.10.1989
(73) Patentinhaber: DENTAURUM J.P. WINKELSTROETER KG, 75228 Ispringen (DE)
(72) Erfinder: Sernetz, Friedrich, Dr., D-7530 Pforzheim (DE)
(74) Vertreter: Hoeger, Stellrecht & Partner

(56) Entgegenhaltungen:
- WO-A-89/08085
- DE-A- 2 163 889
- US-A- 3 562 371
- US-A- 4 323 545

## Beschreibung

Die Erfindung betrifft ein Verfahren zur Herstellung von orthodontischen Hilfsteilen, insbesondere Brackets und Bukkalröhrchen aus transparentem, polykristallinem Aluminiumoxid, wobei in einem ersten Schritt unter Verwendung von reinem, feinkörnigem Aluminiumoxid mit einem Reinheitsgrad >99,0 % ein Grünling gebildet wird, wobei gegebenenfalls in einem zweiten Schritt der Grünling von den bei der Herstellung verwendeten Hilfsmitteln durch Ausbrennen befreit wird, an den sich eine Vorbehandlung, bei der eine geschlossene Formkörperoberfläche erzeugt wird, als dritter Schritt anschließt, und wobei in einem vierten Schritt die Verdichtung zum transparenten, polykristallinen Aluminiumoxid erfolgt, bei der die Restporosität im Innern des Formkörpers geschlossen wird, wobei ein allseitiger Druck bei erhöhter Temperatur angewendet wird.

Ein solches Verfahren ist aus der US-PS 3 562 371 bekannt. Es wird hierbei empfohlen, im dritten Schritt in einer Wasserstoffatmosphäre oder noch besser im Vakuum zu arbeiten, um sicherzustellen, daß keine Gasanteile mehr in dem dichtgesinterten Formkörper enthalten sind. Dies ergibt sich automatisch, wenn im Vakuum gearbeitet wird, da bereits mit der Evakuierung sämtliche Gasanteile aus dem Formkörper entfernt werden, und sobald die geschlossene Oberfläche erreicht wird, ist ein erneutes Eindringen von Gasen, sofern sie nicht im Material des Formkörpers löslich sind und/oder durch diesen hindurchdiffundieren können, nicht mehr möglich. Im Falle, daß mit einer Wasserstoffatmosphäre gearbeitet wird, werden die Gasanteile in dem Formkörper ausgetauscht und es verbleibt in den inneren Poren des Formkörpers nach der Schließung der Porosität an der Oberfläche nur noch ein Gasanteil übrig, der im vierten Schritt der Dichtsinterung aufgrund der Löslichkeit des Wasserstoffs in der Aluminiumoxidmatrix nicht als eingeschlossenes Gasvolumen im Formkörper verbleibt.

In den Grundzügen verwandte Verfahren beschreiben R. L. Coble (J. Amer. Ceram. Soc. 45, 123-127 (1962) sowie die US-PS 30 26 210. Bei diesen Verfahren wird Aluminiumoxid mit bis zu 0,5 % Magnesiumoxid dotiert, um bei der Sinterung ein ausgeprägtes, ungewolltes Kornwachstum zu verhindern, und um damit die Transparenz des gesinterten Materials zu verbessern. Üblicherweise wurde gemäß diesen Verfahren ein vorverdichteter Grünling in sauerstoffhaltiger Atmosphäre im Temperaturbereich von 1000 bis 1200° C vorgesintert und anschließend in Wasserstoffatmosphäre oder im Vakuum bei Temperaturen zwischen 1700 und 1950° C dichtgesintert.

Aus der US-PS 38 99 560 ist ein Verfahren zur Herstellung von transparentem, polykristallinem Aluminiumoxid bekannt, bei dem allerdings eine Reinheit des Aluminiumoxids von mindestens 99,97 % erforderlich ist und bei dem bei Temperaturen oberhalb von 1800° C die Formkörper heiß geschmiedet werden. Eine Dotierung ist bei diesem Verfahren nicht notwendig, jedoch sind wegen dem Heißschmiedeprozeß keine komplexen Formkörperstrukturen erzielbar, wie dies insbesondere bei orthodontischen Teilen wünschenswert ist.

Nach dem gegenwärtigen Stand der Lehrmeinung (vgl. E. Dörre, H. Hübner "Alumina" Springer Verlag 1984, Seite 229 ff.) ist es bei der Herstellung von polykristallinem, transparentem Aluminiumoxid wichtig, die Restporosität in dem Formkörper zu beseitigen, um die Zahl der reflektierenden und damit streuenden Grenzflächen in dem Produkt zu minimieren.

Aus der DE-A-2 163 889 ist ein Verfahren zur Herstellung von transparenten Al₂O₃-Werkstoffen, die für Lampenumhüllungen eingesetzt werden, bekannt, bei dem durch eine Vorbehandlung und eine anschließende isostatische Heißpreßbehandlung ein Produkt mit geschlossener Porosität erhalten wird.

Bei den bisherigen Verfahren wurde dieses in der Weise erreicht, daß bei der Vorsinterung relativ niedrige Temperaturen, d.h. Temperaturen im Bereich von 1000 bis 1200° C gewählt wurden, um eine offene Struktur der Poren bis zur Oberfläche hin zu erhalten, so daß sich in dem vorgesinterten Formkörper eine durchgehende und offene Porenstruktur erhält. In dem zweiten Sinterprozeß, auch Dichtsinterung genannt, wird dann bei sehr hoher Temperatur, d.h. oberhalb von 1700° C, gearbeitet.

Wurde dieser Hochtemperatursinterungsprozeß in Gegenwart von Luft, d.h. in einer im wesentlichen stickstoffhaltigen Atmosphäre, durchgeführt, so blieb eine Restporosität im Aluminiumoxid zurück, was mit der Unlöslichkeit von Stickstoff und sonstigen Inertgasen in Aluminiumoxid erklärt wurde. Deshalb wurde die bisherige Dichtsinterung entweder in einer Wasserstoffatmosphäre vorgenommen, da Wasserstoff eine begrenzte Löslichkeit in Aluminiumoxid aufweist, oder aber im Vakuum, so daß sich bei der Dichtsinterung keine Gaseinschlüsse in dem Aluminiumoxidformkörper bilden konnten.

Diese Verfahren haben jedoch sämtlich den Nachteil, daß die Schlußsinterung bei sehr hohen Temperaturen erfolgen muß. Wegen den erforderlichen hohen Temperaturen und der entweder geforderten Wasserstoffatmosphäre oder dem notwendigen Vakuum sind sehr aufwendige und teure Ofeneinrichtungen notwendig, die das Verfahren insgesamt unwirtschaftlich und nicht allgemein anwendbar machen.

Aufgabe der Erfindung ist es, ausgehend von der Lehre der US-PS 3 562 371 ein Verfahren vorzuschlagen, mit dem orthodontische Hilfsteile, insbesondere Brackets und Bukkalröhrchen als Aluminiumoxidformkörper herstellbar sind, denen gezielt eine Farbtönung verliehen werden kann.

Diese Aufgabe wird bei einem Verfahren der eingangs beschriebenen Art erfindungsgemäß dadurch gelöst, daß bei der Vorbehandlung eine Gasmenge in dem Formkörper eingeschlossen wird, die bei der Verdichtung des Formkörpers im vierten Schritt mit der umgebenden Atmosphäre nicht austauschbar ist und als eingeschlossenes Gasvolumen in dem Formkörper verbleibt, derart, daß in einem nachfolgenden Schritt bei den erhaltenen Formkörpern mittels Temperung an Luft im Temperaturbereich oberhalb von 600° C eine gelbliche Farbtönung erzielbar ist.

Die geschlossene Formkörperoberfläche ist für normale sauerstoffhaltige Atmosphären wie,beispielsweise Luft, als gasdicht anzusehen, so daß die in dem Formkörper eingeschlossene Gasmenge nicht mehr wie bei dem Verfahren nach Coble bei der Dichtsinterung nach außen einfach entweichen bzw. ausgetauscht werden kann.

Das erfindungsgemäße Verfahren wendet sich ganz bewußt von der bisherigen Verfahrensweise ab, bei der besonders darauf geachtet wurde, daß bei der Vorbehandlung Formkörper erhalten wurden, die eine offene Porosität aufweisen, d.h. daß bei der Vorbehandlung den Poren im Innern des Formkörpers noch eine Verbindung nach außen zur Formkörperoberfläche hin erhalten bleibt, und verlangt die Erzielung einer geschlossenen Oberfläche des Formkörpers. Angesichts dieser geschlossenen Formkörperoberfläche ist die Atmosphäre, in der die Dichtsinterung im vierten Schritt erfolgt, im wesentlichen ohne Belang, so daß man die den Formkörper umgebende Gasatmosphäre rein nach Gesichtspunkten aussuchen kann, die für die verwendete Apparatur am vorteilhaftesten ist.

Bei bevorzugten Verfahren wird die Vorbehandlung des Grünlings einen Vorsinterprozeß umfassen.

Der Vorsinterprozeß wird vorzugsweise so durchgeführt, daß sich möglichst kurze Haltezeiten in dem Vorsintertemperaturbereich ergeben, um so ein Kornwachstum im Formkörper auf ein Minimum zu beschränken.

Als besonders günstig hat sich eine Vorbehandlung erwiesen, bei der die Verschmelzung der Oberfläche mittels Laserlicht hergestellt wird, da hier ganz gezielt nur auf den Oberflächenbereich des Formkörpers eingewirkt werden kann und bei dem in dem behandelten Oberflächenbereich eine sprunghafte Temperaturerhöhung erzielbar ist. Auch bei dieser Verfahrensführung ist das unerwünschte Kornwachstum insbesondere auch im Innern des Formkörpers auf ein Minimum reduziert.

Ein weiterer Vorteil des erfindungsgemäßen Verfahrens ergibt sich dadurch, daß nunmehr Aluminiumoxid ohne Dotierung verwendet werden kann. Selbstverständlich lassen sich jedoch auch Aluminiumoxidpulver, denen Dotierungsmaterial beigemengt wurde, mit dem erfindungsgemäßen Prozeß verarbeiten.

Das Aluminiumoxidpulver selbst wird bevorzugt mit einem Reinheitsgrad ≧ 99,7 % verwendet.

Weiter bevorzugt sind Aluminiumoxidpulver mit einem Reinheitsgrad ≧ 99,9 %, bei dem sich die besten Ergebnisse bezüglich der Transparenz ergeben.

Die Temperaturen für die Vorsinterung des Grünlings liegen für den Vorsinterprozeß vorzugsweise oberhalb von 1400° C, wobei der Aufheizprozeß des Grünlings zunächst langsam auf eine Temperatur von ca. 500° C erfolgt, um die bei der Bildung des Grünlings gegebenenfalls verwendeten Hilfsmittel aus dem Grünling auszubrennen. Nach vollständiger Ausbrennung des Hilfsmittels aus dem Grünling wird dann die eigentliche Vorsinterung eingeleitet, indem die Temperatur möglichst schnell auf die Sintertemperatur erhöht wird. Anstelle der Vorsinterung kann auch, wie zuvor erwähnt, die Verschmelzung der Formkörperoberfläche mit Laserlicht treten.

Der bevorzugte Temperaturbereich für die Vorsinterung, in dem in relativ kurzer Zeit die geforderte, geschlossene Porosität an der Oberfläche des Formkörpers erreicht wird, liegt im Bereich von 1500° C bis 1700° C.

Die Vorsinterung des Grünlings wird vorzugsweise in Gegenwart einer sauerstoffhaltigen Atmosphäre, insbesondere Luft, durchgeführt. Bevorzugt werden hierbei Verfahren, bei denen die Aufheizung auf die eigentliche Sintertemperatur möglichst mit ≧ 30° C/min erfolgt, wobei dann die Sintertemperatur für ca. 30 Minuten gehalten wird.

Bei einem bevorzugten Verfahren gemäß der Erfindung wird die Verdichtung des Formkörpers im dritten Verfahrensschritt mittels eines heißisostatischen Preßverfahrens erreicht.

Alternativ dazu ist denkbar, die druckunterstützte Sintermethode (pressure assisted sintering oder PAS) zu verwenden, die ebenfalls bei höheren Temperaturen arbeitet, jedoch Drücke bis maximal 100 bar verwendet.

Beide Verfahren sind in dem erfindungsgemäßen Verfahren gleichermaßen einsetzbar, wobei jedoch das Pressure Assisted Sintering den Vorteil hat, daß die dazu notwendige Ausrüstung wesentlich kleinere Kosten verursacht, da diese Ausrüstung keinen extremen Drücken standhalten muß.

Ein großer Vorzug des erfindungsgemäßen Verfahrens gegegenüber den aus dem Stand der Technik bekannten Verfahren ist darin zu sehen, daß die Temperatur bei dem heißisostatischen Preßverfahren lediglich oberhalb 1000° C liegen muß, wobei bereits für viele Anwendungsbereiche eine zufriedenstellende Transparenz der Formkörper erreicht wird, so z.B. bei orthodontischen Brackets, bei denen eine gewisse Restopakheit durchaus erwünscht ist, um die Formkörper in ihrem Aussehen der Zahnfarbe anzunähern.

Oberhalb 1400° C wird bereits eine ausgezeichnete Transparenz erhalten.

Am meisten bevorzugt sind jedoch Temperaturen für die Dichtsinterung während dem heißisostatischen Preßverfahren im Bereich von 1500° C bis 1700° C, da hier mit kleineren Haltezeiten der Dichtsintertemperatur gearbeitet werden kann.

Zweckmäßigerweise wird das heißisostatische Preßverfahren unter einer Schutzgasatmosphäre durchgeführt. Die Schutzgasatmosphäre ist hierbei nicht für den Formkörper von Bedeutung, da dieser auch in einer sauerstoffhaltigen Atmosphäre dichtgesintert werden könnte, sondern für das Material des Autoklaven bzw. ganz allgemein der verwendeten Dichtsinterapparatur. Demgegenüber ist der Stand der Technik immer auf eine Schutzgasatmosphäre, insbesondere eine reduzierende Atmosphäre wie z.B. Wasserstoffgas, angewiesen, da dort von Wichtigkeit ist, daß die Sinteratmosphäre Gase enthält, die zumindest eine teilweise Löslichkeit in dem Formkörpermaterial aufweisen.

Der Gasdruck wird beim Dichtsintern vorzugsweise ca. 1000 bar betragen.

Wie zuvor erwähnt, läßt sich jedoch das Dichtsintern auch mit der Pressure-Assisted-Sintering-Methode durchführen, bei der lediglich Drücke bis zu 100 bar angewendet werden müssen.

Bei einem bevorzugten Verfahren wird das Ausgangsmaterial für die Formung der Grünlinge aus feinkörnigem Aluminiumoxidpulver gebildet, das eine mittlere Korngröße kleiner als 5 µm aufweist, und das mit einem Bindemittel und gegebenenfalls mit Dotierungsmaterial versetzt und zu einem Granulat mit einer mittleren Granulatkorngröße von ca. 100 µm bis 300 µm sprühgetrocknet wurde. Die mittels dieses Verfahrens hergestellten Formkörper besitzen eine gute Transparenz im sichtbaren Bereich sowie dem nahen UV und nahen IR.

Über die Wahl der Korngröße des Aluminiumoxidpulvers läßt sich in gewisser Weise Einfluß auf den Wellenlängenbereich nehmen, in dem das erfindungsgemäße polykristalline Aluminiumoxid seine maximale Transparenz aufweist.

Das so erhaltene Granulat wird beispielsweise zu den gewünschten Formkörpern einaxial verpreßt.

Nach einer der zuvor beschriebenen Vorbehandlungen sind die polykristallinen Formkörper noch völlig opak, während sich die Transparenz erst nach der Dichtsinterung ergibt, bei der eine Schließung der Restporosität erfolgt.

Das Ergebnis dieses Verfahrens ist in mehrfacher Hinsicht überraschend, da
a) keine Dotierungen des Aluminiumoxids zur Verhinderung des Kornwachstums notwendig sind;
b) da kein Vakuum oder eine reduzierende Atmosphäre wie beispielsweise Wasserstoff zur Vermeidung der Restporosität erforderlich ist und
c) bei deutlich niedrigeren Temperaturen unter Anwendung von allseitigem (Gas)-Druck eine Verdichtung erreicht werden kann, bei der die vorhandene Restporosität geschlossen wird und somit eine Transparenz der Hilfsteile erreicht wird.

Insbesondere wegen den wesentlich niedrigeren Temperaturen bei der Dichtsinterung und wegen dem Verzicht auf ein Vakuum oder eine reduzierende Atmosphäre in der Dichtsinterapparatur steht mit dem erfindungsgemäßen Verfahren eine wesentlich wirtschaftlichere und sicherere Methode zur Erzeugung von transparentem, polykristallinem Aluminiumoxid zur Verfügung als bisher. Ferner ist mit dem erfindungsgemäßen Verfahren eine Möglichkeit geschaffen, ohne Dotierungsmaterial Hilfsteile von komplexer Struktur aus transparentem, polykristallinem Aluminiumoxid herzustellen.

Die mit dem erfindungsgemäßen Verfahren erhaltenen orthodontischen Hilfsteile, im folgenden kurz Formkörper genannt, sind auch in weiten Temperaturbereichen einsetzbar. Erst ab Temperaturen oberhalb 600° C ergeben sich leichte Veränderungen, die allerdings nicht die Transparenz des Formkörpers betreffen, sondern vielmehr seine Farbe. In diesem Temperaturbereich beginnen die Formkörper je nach Temperatur und Behandlungszeit eine leichte gelbliche Tönung anzunehmen. Diese Farbveränderung kann sehr gezielt herbeigeführt werden und wirkt sich insbesondere bei Formkörpern für den Dentalbereich vorteilhaft aus, da diese damit in ihrer Farbe der Zahnfarbe angenähert werden können.

Die Stabilität der Transparenz der erfindungsgemäß hergestellten Formkörper zeigt sich daran, daß diese ihre Transparenz bis ca. 1200° C nicht verlieren, so daß ein Temperaturbereich für das Erzielen einer Farbtönung von 600°C bis 1200° C nutzbar ist.

Diese und weitere Vorteile werden im folgenden an Hand eines Verfahrensbeispiels noch näher beschrieben:

Handelsübliches Aluminiumoxidpulver der Reinheit 99,9 % mit einer mittleren Korngröße von kleiner als 2 µm wird mit 0,05 Gew. % Magnesiumoxid, 0,05 Gew. % Yttriumoxid und als Bindemittel mit 1 Gew. % Polyvinylalkohol vermischt und zu Granulat sprühgetrocknet, wobei eine Granulatkorngröße im Bereich von ca. 200 µm entsteht. Die Granulatgröße wird zwar bevorzugt im Bereich von 100 bis 300 µm gehalten, ist jedoch für das Verfahren selbst nicht essentiell.

Ein solchermaßen dotiertes und mit Preßhilfsmitteln versehenes Aluminiumoxidgranulat wird in einer Presse einaxial zu den Formkörpern verdichtet, wobei neben dem einaxialen Kaltpressen auch ein Isostatpressen, Spritzgießen oder Extrudieren in Frage kommt.

Dem anschließenden Vorsinterungsprozeß wird zunächst eine Periode mit langsamer Erwärmung vorgeschaltet, bei der die Formkörper an der Luft auf ca. 500° C in Aufwärmzeiten, die je nach Größe der Formkörper typisch zwischen 2 bis 10 Stunden betragen, aufgeheizt werden. Während dieser Zeit sind die Poren in dem Grünling offen und bilden ein im wesentlichen durchgehendes Porenvolumen. Am Ende der Vorheizzeit ist sämtliches Preßhilfsmittel aus dem Formkörper oder Grünling ausgebrannt, so daß nur noch der reine Aluminiumoxidformkörper, gegebenenfalls mit der Dotierung, vorhanden ist.

Daran schließt sich nun die eigentliche Vorsinterung an, die ebenfalls in Gegenwart von Luftsauerstoff durchgeführt werden kann, wobei die Formkörper in möglichst kurzer Zeit, d.h. bevorzugt mit einer Aufheizgeschwindigkeit von > 30° C/min auf eine Vorsintertemperatur von 1600° C erhitzt werden.

Bei dieser Temperatur reichen relativ kurze Haltezeiten von ca. 30 Minuten aus, um eine Verdichtung an der Oberfläche mit geschlossener Porosität zu erzeugen. Die Temperatur von 1600° C ist nicht absolut notwendig, da bereits bei wesentlich geringeren Temperaturen, d.h. schon oberhalb von 1400° C, eine solche geschlossene Porosität an der Formkörperoberfläche erreicht werden kann. Allerdings verlängern sich bei diesen niedrigeren Temperaturen dann die Haltezeiten für die Vorsintertemperatur.

Diese vorgesinterten Teile werden anschließend in einer handelsüblichen Heißisostatpresse bei Temperaturen von vorzugsweise 1600° C für ca. 30 Minuten bei einem Druck von ca. 1000 bar nachverdichtet, wobei sich die Restporosität schließt. Beim Heißisostatpressen sollte die Aufheizzeit wiederum möglichst kurz gewählt werden, d.h. kleiner als eine Stunde sein, um ein unerwünschtes Kornwachstum zu verhindern.

Bei diesem Verfahren ist zur Verhinderung von unerwünschtem Kornwachstum eine Dotierung des Aluminiumoxidmaterials nicht zwingend notwendig, um eine zufriedenstellende Transparenz des Formkörpermaterials zu erreichen.

Durch die höhere Vorsintertemperatur, bei der eine geschlossene Porosität an der Oberfläche des Formkörpers erreicht wird, die so weit geht, daß die Formkörperoberfläche für Gase wie z.B. Sauerstoff oder Stickstoff gasdicht ist, kann sich die eigentliche Dichtsinterung in der Heißisostatpresse auf eine wesentlich niedrigere Temperatur beschränken, als sie nach den Verfahren gemäß dem Stand der Technik notwendig ist.

Wegen der wesentlich kleineren Temperaturen bei der Dichtsinterung ist auch während dieses Prozesses die Kornwachstumsgeschwindigkeit wesentlich verringert, so daß insbesondere aus diesen Gründen die sonst zwingend notwendigen Dotierungsmittel problemlos entfallen können.

## Patentansprüche

1. Verfahren zur Herstellung von orthodontischen Teilen, insbesondere Brackets und Bukkalröhrchen aus transparentem, polykristallinem Aluminiumoxid, wobei in einem ersten Schritt unter Verwendung von reinem, feinkörnigem Aluminiumoxid mit einem Reinheitsgrad >99,0 % ein Grünling gebildet wird, wobei gegebenenfalls in einem zweiten Schritt der Grünling von den bei der Herstellung verwendeten Hilfsmitteln durch Ausbrennen befreit wird, an den sich eine Vorbehandlung, bei der eine geschlossene Formkörperoberfläche erzeugt wird, als dritter Schritt anschließt, und wobei in einem vierten Schritt die Verdichtung zum transparenten, polykristallinen Aluminiumoxid erfolgt, bei der die Restporosität im Innern des Formkörpers geschlossen wird, wobei ein allseitiger Druck bei erhöhter Temperatur angewendet wird, dadurch gekennzeichnet, daß bei der Vorbehandlung eine Gasmenge in dem Formkörper eingeschlossen wird, die bei der Verdichtung des Formkörpers im vierten Schritt mit der umgebenden Atmosphäre nicht austauschbar ist und als eingeschlossenes Gasvolumen in dem Formkörper verbleibt, derart, daß in einem nachfolgenden Schritt bei den erhaltenen Formkörpern mittels Temperung an Luft im Temperaturbereich oberhalb 600°C eine gelbliche Farbtönung erzielbar ist.

2. Verfahren nach Anspruch 1, dadurch gekennzeichnet, daß bei der Vorbehandlung die Oberfläche mittels Laserlicht verschmolzen wird.

3. Verfahren nach Anspruch 1, dadurch gekennzeichnet, daß die erhaltenen Formkörper zur Erzielung einer gelblichen Farbtönung in einem weiteren Verfahrensschritt einer Temperung an Luft im Temperaturbereich oberhalb 600°C unterworfen werden.

4. Verfahren nach einem der Ansprüche 1 bis 2, dadurch gekennzeichnet, daß die Vorbehandlung eine Vorsinterung umfaßt.

5. Verfahren nach einem der Ansprüche 1 bis 4, dadurch gekennzeichnet, daß dem reinen Aluminiumoxid bei der Grünlingherstellung ein Dotierungsmittel zugegeben wird.

6. Verfahren nach einem der Ansprüche 4 oder 5, dadurch gekennzeichnet, daß die Vorsinterung bei einer Temperatur oberhalb von 1400° C durchgeführt wird.

7. Verfahren nach einem der voranstehenden Ansprüche, dadurch gekennzeichnet, daß die Vorsinterung in Gegenwart einer sauerstoffhaltigen Atmosphäre durchgeführt wird.

8. Verfahren nach einem der voranstehenden Ansprüche, dadurch gekennzeichnet, daß die Aufheizung auf die Sintertemperatur mit mindestens 30°C pro Minute erfolgt.

9. Verfahren nach einem der voranstehenden Ansprüche, dadurch gekennzeichnet, daß die Verdichtung im vierten Verfahrensschritt mittels eines heißisostatischen Preßverfahrens erreicht wird.

10. Verfahren nach Anspruch 9, dadurch gekennzeichnet, daß die Temperatur beim heißisostatischen Preßverfahren oberhalb von 1400° C gehalten wird.

11. Verfahren nach einem der Ansprüche 9 oder 10, dadurch gekennzeichnet, daß das heißisostatische Preßverfahren unter Schutzgasatmosphäre durchgeführt wird.

12. Orthodontische Brackets aus transparentem, polykristallinem Aluminiumoxid, erhalten durch ein Verfahren gemäß einem der voranstehenden Ansprüche.

## Claims

1. A process for the manufacture of orthodontic parts, in particular brackets and buccal tubes made from transparent, polycrystalline aluminium oxide, whereby in a first step a green compact is formed by using pure, fine-grain aluminium oxide having a degree of purity > 99.0 %, whereby if required in a second step the green compact is liberated from the aids used during manufacture by baking, after which a preliminary treatment, in which a closed moulded body surface is produced, follows as a third step, and whereby in a fourth step densification occurs to produce the transparent, polycrystalline aluminium oxide, in which the residual porosity is enclosed inside the moulded body, whereby a universal pressure at elevated temperatures is applied,
**characterised in that** during the preliminary treatment a quantity of gas is enclosed in the moulded body and during the densification of the moulded body in the fourth step it cannot be exchanged with the surrounding atmosphere and remains as an enclosed gas volume in the moulded body so that in a subsequent step with the moulded bodies obtained a yellowish shade can be achieved by means of annealing in air in the temperature range above 600°C.

2. A process according to Claim 1,
**characterised in that** during the preliminary treatment the surface is melted by means of laser light.

3. A process according to Claim 1,
**characterized in that** in a further process step the moulded bodies obtained undergo annealing in air in the temperature range of above 600°C to achieve a yellowish shade.

4. A process according to one of Claims 1 to 2,
**characterized in that** the preliminary treatment comprises a presintering process.

5. A process according to one of Claims 1 to 4,
**characterised in that** a doping agent is added to the pure aluminium oxide during the manufacture of the green compact.

6. A process according to one of Claims 4 or 5,
**characterized in that** presintering is performed at a temperature of above 1400°C.

7. A process according to one of the preceding Claims,
**characterized in that** presintering is performed in the presence of an oxygenous atmosphere.

8. A process according to one of the preceding Claims,
**characterized in that** heating to sintering temperature occurs by at least 30°C per minute.

9. A process according to one of the preceding Claims,
**characterized in that** densification in the fourth process step is achieved by means of a high-temperature isostatic pressing process.

10. A process according to Claim 9,
**characterised in that** the temperature during the high-temperature isostatic pressing process is kept above 1400°C.

11. A process according to one of Claims 9 or 10,
**characterized in that** the high-temperature isostatic pressing process is performed in a controlled atmosphere.

12. Orthodontic brackets made from transparent, polycrystalline aluminium oxide, obtained by a process according to one of the preceding Claims.

## Revendications

1. Procédé de fabrication de pièces orthodontiques, en particulier agrafes et bagues buccales, en oxyde d'aluminium polycristallin et transparent, procédé dans lequel on forme en une première opération une ébauche en utilisant de l'oxyde d'aluminium pur et à grains fins avec un degré de pureté >99,0%, on libère le cas échéant en une seconde opération par cuisson l'ébauche des adjuvants utilisés pour la préparation, et on procède ensuite à un pré-traitement, en tant que troisième opération, au cours duquel on produit une surface fermée pour le corps, et on effectue en une quatrième opération le compactage en oxyde d'aluminium polycristallin et transparent, au cours duquel la porosité résiduelle à l'intérieur du corps est fermée, en utilisant une pression polydirectionnelle et à température élevée, caractérisé en ce que l'on enferme lors du pré-traitement une quantité de gaz dans le corps, qui ne peut être échangée avec l'atmosphère environnant lors du compactage du corps au cours de la quatrième opération, et reste dans le corps sous la forme de volumes gazeux enfermés, de telle manière que l'on peut obtenir une coloration jaunâtre lors d'une opération suivante dans le corps obtenu, au moyen d'un recuit à l'air dans la plage de température supérieure à 600°C.

2. Procédé selon la revendication 1, caractérisé en ce que lors du pré-traitement, on fait fondre la surface au moyen d'une lumière laser.

3. Procédé selon la revendication 1, caractérisé en ce que les corps obtenus sont soumis au cours d'une autre opération de procédé à un recuit à l'air dans la plage de température supérieure à 600°C, afin d'obtenir une coloration jaunâtre.

4. Procédé selon l'une queleonque des revendications 1 à 2, caractérisé en ce que le pré-traitement comprend un pré-frittage.

5. Procédé selon l'une quelconque des revendications 1 à 4, caractérisé en ce que l'on ajoute un produit de dopage à l'oxyde d'aluminium pur lors de la réalisation de l'ébauche.

6. Procédé selon l'une quelconque des revendications 4 ou 5, caractérisé en ce que le pré-frittage est mené à une température supérieure à 1400°C.

7. Procédé selon l'une quelconque des revendications précédentes, caractérisé en ce que le pré-frittage est mené en présence d'une atmosphère contenant de l'oxygène.

8. Procédé selon l'une quelconque des revendications précédentes, caractérisé en ce que le réchauffage jusqu'à la température de frittage se produit à au moins 30°C par minute.

9. Procédé selon l'une quelconque des revendications précédentes, caractérisé en ce que le compactage est atteint au cours de la quatrième opération de procédé au moyen d'un procédé de pressage isostatique à chaud.

10. Procédé selon la revendication 9, caractérise en ce que la température est maintenue au-dessus de 1400°C au cours du procédé de pressage isostatique.

11. Procédé selon l'une ou l'autre des revendications 9 et 10, caractérisé en ce que le procédé de pressage isostatique à chaud est mené sous une atmosphère de gaz protecteur.

12. Agrafes orthodontiques en oxyde d'aluminium polycristallin et transparent, obtenues au moyen d'un procédé selon l'une quelconque des revendications précédentes.
